# FASCICULE DE BREVET EUROPEEN

(11) **EP 1 414 404 B1**
(45) Date de publication et mention de la délivrance du brevet: **29.03.2006**
(21) Numéro de dépôt: 02774856.5
(22) Date de dépôt: 29.07.2002
(51) Int. Cl.: A61K 8/97, A61Q 19/08

(54) **UTILISATION D'UN EXTRAIT DE NOYAUX DE DATTES POUR TRAITER LES MANIFESTATIONS DU VIEILLISSEMENT CUTANE**
VERWENDUNG VON DATTELKERN-EXTRAKT ZUR MINDERUNG DER ANZEICHEN DER HAUTALTERUNG
USE OF DATE STONE EXTRACT FOR TREATING THE SIGNS OF AGING SKIN

(30) Priorité: 07.08.2001 FR 0110528
(43) Date de publication de la demande: 06.05.2004
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2002/002718
(87) Numéro de publication internationale: WO 2003/013463

(56) Documents cités:
- DE-C- 3 442 961
- FR-A- 2 687 310
- FR-A- 2 712 601
- US-A- 5 985 300
- "L'ami des ingrédients naturels-L'encyclopédie" 28 février 1997 (1997-02-28) , ALBAN MULLER XP002197210 voir Dattier, paragraphe Fonction
- DATABASE WPI Week 199748 Derwent Publications Ltd., London, GB; AN 1997-521875 XP002197212 & JP 09 249578 A (YAKURIGAKU CHUO KENKYUSHO KK)
- DATABASE CAPLUS [en ligne] CHEMICAL ABSTRACTS SERVICE, COLUMBUS, OHIO, US; retrieved from STN Database accession no. 2000:600813 XP002197211 & CN 1 243 819 A (PEOP. REP. CHINA) 9 février 2000 (2000-02-09)

## Description

La présente invention se rapporte à l'utilisation non-thérapeutique d'une quantité efficace d'un extrait de noyaux de dattes, sous forme d'une composition cosmétique, pour traiter les manifestations cutanées du vieillissement, pour diminuer les rides et/ou les ridules, ou pour lisser la peau.

La recherche très active entreprise ces dernières décennies sur le traitement du vieillissement s'oriente selon plusieurs axes (matrice extra-cellulaire, stimulation cellulaire, énergie cellulaire, activation de molécules cibles...). Cependant, pour les consommateurs, le marqueur du vieillissement demeure la ride. De nombreux produits proposent de diminuer les rides, mais à ce jour, peu de résultats sont significatifs.

On constate donc que subsiste le besoin d'un produit nouveau, ayant une action sur les rides.

Or, la demanderesse a trouvé, de façon surprenante et inattendue, qu'une quantité efficace d'un extrait de noyaux de dattes a une action sur la diminution des rides.

A la connaissance de la demanderesse, il n'a jamais été décrit dans l'art antérieur l'utilisation d'un extrait de noyaux de dattes pour diminuer les rides.

Ainsi, l'invention a pour objet l'utilisation non-thérapeutique d'une quantité efficace d'un extrait de noyaux de dattes, sous forme d'une composition cosmétique, pour traiter les manifestations cutanées du vieillissement, pour diminuer les rides et/ou les ridules, ou pour lisser la peau.

L'homme de l'art, connaissant le métier de l'extraction et de la purification des végétaux, pourra aisément envisager d'autres procédés d'obtention de l'extrait décrit par la demanderesse.

De plus, l'extraction peut être remplacée par des techniques de macération, décoction, lixiviation, extraction sous reflux, ultrasons, micro-ondes, fluide super-critique.

La composition selon l'invention est préférentiellement une composition cosmétique et/ou dermatologique adaptée à l'administration par voie topique cutanée comprenant un milieu cosmétiquement ou dermatologiquement acceptable.

Dans les compositions de l'invention, l'extrait est utilisé sous forme pure ou en solution dans un solvant adapté à la cosmétique.

Selon un mode de réalisation avantageux de l'invention, l'extrait est préalablement solubilisé dans un ou plusieurs solvants cosmétiquement acceptables comme le propylène glycol, le butylène glycol, le dipropylène glycol, les diglycols, la vaseline, l'hexyl décanol, les huiles végétales.

Selon encore un autre mode de réalisation avantageux de l'invention, l'extrait de dattes est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

Pour donner un ordre de grandeur, dans les compositions cosmétiques ou dermatologiques de la présente invention, l'extrait est utilisé en une quantité représentant de 0,0001 % à 20 % du poids total de la composition, et préférentiellement en une quantité représentant de 0,001 % à 5 % du poids total de la composition.

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les poils ou les cheveux. Ces compositions pourront notamment se présenter sous forme de crèmes, émulsions huile-dans-eau, eau-dans-huile ou émulsions multiples, solutions, suspensions, ou encore poudres, adaptées à une application sur la peau, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, de façon connue, les adjuvants nécessaires à leur formulation, tels que solvants, épaississants, diluants, anti-oxydants, colorants, filtres, pigments, charges, conservateurs, parfums, absorbeurs d'odeur. Dans tous les cas, ces adjuvants, ainsi que leurs proportions, seront choisis de manière à ne pas nuire aux propriétés recherchées dans l'invention. Ces adjuvants peuvent, par exemple, correspondre à 0,01 à 20 % du poids total de la composition.

Lorsque la composition de l'invention est une émulsion, la phase grasse peut représenter de 5 à 80 % en poids et de préférence de 5 à 50 % en poids par rapport au poids total de la composition. Les émulsionnants et coémulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme de métier veillera à choisir les éventuels composés complémentaires, actifs ou non-actifs, et/ou leur quantité, de telle sorte que les propriétés avantageuses du mélange ne soient pas, ou sensiblement pas, altérées par l'adjonction envisagée.

D'autres avantages et caractéristiques de l'invention apparaîtront mieux à la lecture des exemples donnés à titre illustratif et non limitatif.

### Exemple 1- Préparation d'un extrait de noyaux de dattes

Des noyaux de dattes sont lavés, séchés, broyés, puis plusieurs extractions sont réalisées par exemple avec 10 volumes d'un mélange chloroforme/méthanol 2:1. Cet extrait est utilisé dans les exemples suivants sous le nom « extrait de l'exemple 1 ».
Puis le solvant est évaporé et l'extrait remis en solution à 3 % dans un solvant adapté à la cosmétique, par exemple l'huile de palme.

### Exemple 2 - Effets de l'extrait de l'exemple 1 sur les rides

L'extrait resolubilisé à 3 % dans de l'huile de palme est incorporé dans une crème à 5 %. Un groupe de 10 volontaires applique pendant 5 semaines, 2 fois par jour, la crème contenant l'actif sur une partie du visage et sur l'autre la crème placebo. Après 4 semaines, des observations cliniques sont effectuées ainsi que des empreintes de rides (répliques de silicone).
Les analyses statistiques ont été réalisées en utilisant le test non paramétrique de Wilcoxon pour les séries appariées.

Les résultats *in vivo* montrent une diminution significative des rides et ridules apportée par la crème contenant 0,15 % de l'extrait de noyaux de dattes de l'exemple 1. De ce fait, l'extrait est d'un grand intérêt dans les produits cosmétique de soin anti-âge.

### Exemple 3 - Préparation de compositions

Ces compositions ont été obtenues par simple mélange des différents composants. Les quantités indiquées sont données en pourcentage de poids.

### 1-Emulsion huile dans eau

### Phase huileuse :

■ Montanov 68 (Cetearyl Alcohol and Cetearyl Glucoside) 5.00 %
■ Huile de Palme 5.00 %
■ Extrait de l'exemple 1 0.15 %
■ Huile de Vaseline 5.00 %
■ Isopropyl Palmitate 7.00 %

### Phase aqueuse:

■ Glycérine 5.00 %
■ Allantoïne 0.10 %
■ Sepigel 305 (Polyacrylamide and C13-14 Isoparaffin and Laureth-7) 0.30 %
**■** Conservateur 0.50 %
■ Parfum 0.50 %
■ Eau qsp 100 %

### 2- Gel

■ Carbopol Ultrez 10 (sol. à 2%) 25.00 %
■ Triéthanolamine 0.50 %
**■** Extrait de l'exemple 1 1.00 %
■ Conservateur 0.20 %
■ EDTA (séquestrant) 0.10 %
■ Parfum 0.50 %
■ Eau qsp 100 %

### 3-Lotion

■ Mono Propylène Glycol 1.00 %
**■** Allantoïne 0.30 %
■ Glycérine 1.00 %
■ Cetiol HE (PEG-7 Glyceryl Cocoate) 1.00 %
■ Extrait de l'exemple 1 0.01%
■ Conservateur 0.20 %
■ Parfum 0.50 %
■ Eau qsp 100 %

## Revendications

1. Utilisation non-thérapeutique d'une quantité efficace d'un extrait de noyaux de dattes, sous forme d'une composition cosmétique, pour traiter les manifestations cutanées du vieillissement, pour diminuer les rides et/ou les ridules, ou pour lisser la peau.

2. Utilisation selon la revendication précédente, **caractérisée en ce que** l'extrait est utilisé sous forme pure ou en solution dans un solvant adapté à la cosmétique.

3. Utilisation selon l'une quelconque des revendications précédentes, **caractérisée en ce que** l'extrait est préalablement solubilisé dans un ou plusieurs solvants cosmétiquement acceptables comme le propylène glycol, le butylène glycol, le dipropylène glycol, les diglycols, la vaseline, l'hexyl décanol, les huiles végétales.

4. Utilisation selon l'une quelconque des revendications précédentes, **caractérisée en ce que** l'extrait est préalablement solubilisé dans un vecteur cosmétique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

5. Utilisation selon l'une quelconque des revendications précédentes, **caractérisée en ce que** l'extrait est utilisé en une quantité représentant de 0,0001 % à 20 % du poids total de la composition, et préférentiellement en une quantité représentant de 0,001 % à 5 % du poids total de la composition.

## Patentansprüche

1. Nicht-therapeutische Verwendung einer wirksamen Menge eines Dattelkernextraktes in Form einer kosmetischen Zusammensetzung zur Behandlung von Alterserscheinungen der Haut, zur Verringerung von Falten und/oder Fältchen oder zum Glätten der Haut.

2. Verwendung gemäß dem vorangehenden Anspruch, **dadurch gekennzeichnet, dass** der Extrakt in purer Form oder in Lösung in einem für die Kosmetik angepassten Lösungsmittel verwendet wird.

3. Verwendung gemäß einem der vorangehenden Ansprüche, **dadurch gekennzeichnet, dass** der Extrakt zunächst in einem oder mehreren kosmetisch verträglichen Lösungsmittel(n) wie Propylenglykol, Butylenglycol, Dipropylenglycol, Diglycolen, Vaseline, Hexyldecanol, pflanzlichen Ölen solubilisiert wird.

4. Verwendung gemäß einem der vorangehenden Ansprüche, **dadurch gekennzeichnet, dass** der Extrakt zunächst in einem kosmetischen Träger wie Liposome solubilisiert wird oder auf organischen Polymeren in Pulverform, mineralischen Trägern wie Talken und Bentoniten adsorbiert wird und noch allgemeiner solubilisiert wird in oder fixiert wird auf einem beliebigen Träger der kosmetisch oder pharmazeutisch verträglich ist.

5. Verwendung gemäß einem der vorangehenden Ansprüche, **dadurch gekennzeichnet, dass** der Extrakt in einer Menge verwendet wird, die 0,0001% bis 20% des Gesamtgewichts der Zusammensetzung entspricht, und bevorzugt in einer Menge, die 0,001 % bis 5% des Gesamtgewichts der Zusammensetzung entspricht.

## Claims

1. Non-therapeutic use of an effective quantity of a date stone extract in the form of a cosmetic composition for treating the cutaneous manifestations of ageing, for reducing lines and/or wrinkles, or for smoothing the skin.

2. Use according to the preceding claim, **characterised in that** the extract is used in pure form or in solution in a solvent suitable for cosmetics.

3. Use according to any one of the preceding claims, **characterised in that** the extract is previously solubilised in one or more cosmetically acceptable solvents such as propylene glycol, butylene glycol, dipropylene glycol, diglycols, petroleum jelly, hexyldecanol or vegetable oils.

4. Use according to any one of the preceding claims, **characterised in that** the extract is previously solubilised in a cosmetic carrier such as liposomes or adsorbed onto pulverulent organic polymers, inorganic supports such as talcs and bentonites, and more generally solubilised in or immobilised on any cosmetically or pharmaceutically acceptable carrier.

5. Use according to any one of the preceding claims, **characterised in that** the extract is used in a quantity amounting to from 0.0001% to 20% of the total weight of the composition, and preferably in a quantity amounting to from 0.001% to 5% of the total weight of the composition.
